# EUROPEAN PATENT SPECIFICATION

(11) **EP 1 720 540 B1**
(45) Date of publication and mention of the grant of the patent: **11.06.2008**
(21) Application number: 05715410.6
(22) Date of filing: 18.02.2005
(51) Int. Cl.: A61K 31/282, A61P 35/00, A61K 33/24

(54) **SATRAPLATIN FOR TREATING RESISTANT OR REFRACTORY TUMORS**
SATRAPLATIN ZUR BEHANDLUNG VON RESISTENTEN ODER REFRKATÄREN TUMOREN
SATRAPLATIN DANS LE TRAITEMENT DE TUMEURS RESISTANTES OU REFRACTAIRES

(30) Priority: 18.02.2004 US 546097 P
(43) Date of publication of application: 15.11.2006
(60) Divisional application: 08102968.8
(73) Proprietor: GPC Biotech AG, 82152 Martinsried (DE)
(72) Inventor: CALIGIURI, Maureen, Reading, MA 01867 (US); WOSIKOWSKI-BUTERS, Katja, 85586 Poing (DE); CASAZZA, Anna Maria, Madison, CT 06443 (US)
(74) Representative: Dörries, Hans Ulrich
(86) International application number: PCT/EP2005/001733
(87) International publication number: WO 2005/077385

(56) References cited:
- WO-A-20/04105761
- US-A- 6 025 473
- PLASENCIA CARMEN ET AL: "Antiproliferative effects of ZD0473 (AMD473) in combination with 5-Fluorouracil or SN38 in human colorectal cancer cell lines" INVESTIGATIONAL NEW DRUGS, vol. 22, no. 4, November 2004 (2004-11), pages 399-409, XP002330769 ISSN: 0167-6997
- SOULIE P ET AL: "Oxaliplatin/cisplatin (L-OHP/CDDP) Combination in Heavily Pretreated Ovarian Cancer" EUROPEAN JOURNAL OF CANCER, PERGAMON PRESS, OXFORD, GB, vol. 33, no. 9, August 1997 (1997-08), pages 1400-1406, XP004284356 ISSN: 0959-8049
- ZELEK L ET AL: "Phase II study of oxaliplatin and fluorouracil in taxane- and anthracycline-pretreated breast cancer patients." JOURNAL OF CLINICAL ONCOLOGY : OFFICIAL JOURNAL OF THE AMERICAN SOCIETY OF CLINICAL ONCOLOGY. 15 MAY 2002, vol. 20, no. 10, 15 May 2002 (2002-05-15), pages 2551-2558, XP002330770 ISSN: 0732-183X
- SHARP S Y ET AL: "Lack of a role for MRP1 in platinum drug resistance in human ovarian cancer cell lines" BRITISH JOURNAL OF CANCER, vol. 78, no. 2, July 1998 (1998-07), pages 175-180, XP009048459 ISSN: 0007-0920
- MEDINA-GUNDRUM LETICIA ET AL: "AMD473 (ZD0473) exhibits marked in vitro anticancer activity in human tumor specimens taken directly from patients." ANTI-CANCER DRUGS, vol. 14, no. 4, April 2003 (2003-04), pages 275-280, XP009048496 ISSN: 0959-4973
- SMITH D C: "Chemotherapy for hormone refractory prostate cancer" UROLOGIC CLINICS OF NORTH AMERICA 1999 UNITED STATES, vol. 26, no. 2, 1999, pages 323-331, XP009048474 ISSN: 0094-0143

## Description

### BACKGROUND OF THE INVENTION

Clinical drug resistance is a major barrier to overcome before chemotherapy can become curative for most patients with cancer. In many common cancers (for example, non-small cell lung, testicular and ovarian cancers), substantial tumour shrinkage can be expected in more than 50% of cases with conventional chemotherapy. In other cases, response rates are lower; only 10-20% of patients with renal cell carcinoma, pancreatic and oesophageal cancers respond to treatment. In almost all cases, drug resistance eventually develops shortly and is often fatal. If this could be treated, prevented or surmounted, the impact would be substantial.

Clinical tumour resistance to chemotherapy can be intrinsic or acquired. Intrinsic resistance is present at the time of diagnosis in tumours that fail to respond to first-line chemotherapy. Acquired resistance occurs in tumours that are often highly responsive to initial treatment, but develop resistance in the course of treatment, or on tumour recurrence, exhibiting an entirely different phenotype. They may become resistant to both previously used drugs, and new agents with different structures and mechanisms of action.

Platinum compounds are among the most active chemotherapeutic agent available for the treatment of a variety of malignancies, including testicular and ovarian carcinoma. The use of some of these compounds, e.g., cisplatin, is restricted by both toxological and resistance considerations. To overcome these issues, efforts were started to discover novel platinum compounds which do not share these properties of cisplatin. One compound that was identified is satraplatin (JM216), a platinum (Pt) IV complex. Satraplatin has advantages compared to cisplatin due to its oral availability and favourable safety profile, such as the absence of kidney- and neurotoxicity. Activity of satraplatin has been shown in prostate, ovarian and SCL carcinoma patients. In a Phase II-III clinical trial in Hormone Refractory Prostate Carcinoma (HRPC) patients, the combination of satraplatin plus prednisone was more active than prednisone alone (ASCO, 2003). The current standard treatment of HRPC is primarily palliative and includes 1^{st} line chemotherapeutic regimens with agents such as estramustine, mitoxantrone and taxanes, with docetaxol being increasingly used as a first-line chemotherapeutic agent

Given the prevalence of clinical cancer drug resistance and its dire consequences for cancer patients, it is desirable to have treatments of tumors resistant to a first-line therapeutic agent. Such treatments are provided herein.

### SUMMARY OF THE INVENTION

The present invention relates to the use of a compound of the structure 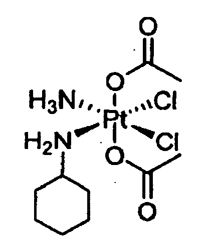 for the preparation of a pharmaceutical composition for the treatment of a cancer or tumor resistant or refractory to a taxane. Said treatment may results in killing or inhibition of the growth of tumor cells.

In one embodiment, the resistance or refractoriness of said cancer or tumor is mediated through tubulin. It may also be mediated through multidrug resistance, e.g., multidrug resistance mediated through overexpression of an ABC transporter.

The taxane in respect of which the cancer or tumor is resistant or refractory may be paclitaxel. Alternatively, it may be docetaxel.

In one embodiment, the tumor resistant or refractory to the taxane comprises a solid tumor. For instance it may be a solid tumor selected from: breast cancer, cervical cancer, colorectal cancer, peritoneal cancer, ovarian cancer, bronchial cancer, small cell lung cancer, non-small cell lung cancer, gastric, prostate, and head and neck cancer, or metastases thereof. Alternatively, the tumor may comprise a hematological tumor.

In one embodiment of the use according to the invention, the treatment includes the further administration of an effective amount of another pharmaceutical ingredient. Said other pharmaceutical ingredient may be an anti-emetic or anti-diarrheal therapeutic composition. It may also be an agent that overcomes a specific drug resistance mechanism. Furthermore, it may be another anti-cancer therapeutic agent.

In certain embodiments, tumors resistant to a taxane include those for which resistance is mediated by multidrug resistance. Such multidrug resistance may be mediated through an ATP-binding cassette (ABC) transporter such as P-glycoprotein (P-gp). Non-platinum-based therapeutic agents for which resistance is mediated through P-gp include: vinca alkaloids (vinblastine), the anthracyclines (adriamycin), the epipodophyllotoxins (etoposide), taxanes (paclitaxel, docetaxel), antibiotics (actinomycin D and gramicidin D), antimicrotubule drugs (colchicine), protein synthesis inhibitors (puromycin), toxic peptides (valinomycin), topoisomerase I inhibitors (topotecan), DNA intercalators (ethidium bromide) and anti-mitotics.

### BRIEF DESCRIPTION OF THE DRAWING

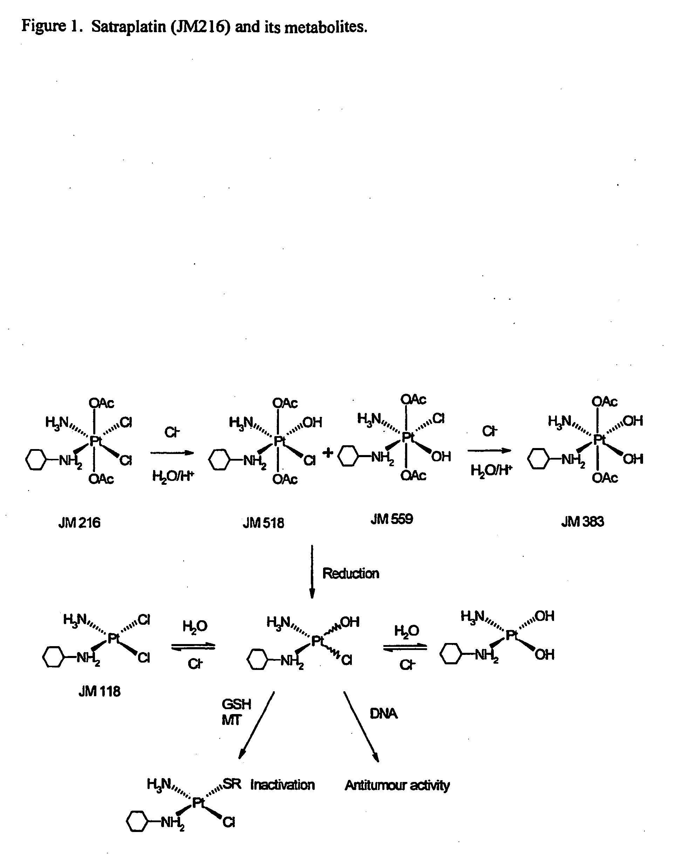

Figure 1. Satraplatin (JM216) and certain of its metabolites according to Raynaud et al 1996.

### DEtAILED DESCRIPTION OF THE INVENTION

### I. Overview

Described herein is the use of satraplatin for the preparation of a pharmaceutical composition for the treatment of a cancer resistant or refractory to taxane. The present invention is based, at least in part, on Applicants' discovery that the effectiveness of satraplatin is maintained in proliferating cells such as tumor cells resistant to taxane. Significantly, Applicants have demonstrated that satraplatin can overcome drug resistance mediated through multiple different mechanisms. Each of these resistance mechanisms confers tumor resistance on a variety of drugs. In light of Applicants' discovery, patients with tumors resistant to a taxane may benefit from treatment according to the use described herein.

### II. Definitions

The terms "administered", "administration", "administering" a compound will be understood to mean providing any compound of the use described herein to an individual in need of treatment.

The term "alkyl" refers to optionally substituted straight- or branched-chain saturated hydrocarbon groups having from 1 to about 20 carbon atoms, preferably from 1 to about 7 carbon atoms. Examples of alkyl include, but are not limited to, methyl, ethyl, npropyl, i-propyl, n-butyl, s-butyl, t-butyl, n-pentyl, and s-pentyl. In addition, the term is intended to include both unsubstituted and substituted alkyl groups, the latter referring to alkyl moieties having one or more hydrogen substituents replaced by, but not limited to halogen, hydroxyl, carbonyl, alkoxy, ester, ether, cyano, phosphoryl, amino, amino, amido, sulfhydryl, alkythio, thioester, sulfonyl, nitro, heterocyclo, aryl or heteroaryl. It will also he understood by those skilled in the art that the substituted moieties themselves can be substituted as well when appropriate.

The term "cycloalkyl" refers to optionally substituted saturated cyclic hydrocarbon ring systems, preferably containing 3 to 7 carbons per ring. Exemplary groups include cyclopropyl, cyclobutyl, cyclopentyl, cyclohexyl, cycloheptyl, cyclooctyl, cyclodecyl, cyclododecyl, and adamantyl. Exemplary substituents include one or more alkyl groups as described above, or one or more of the groups described above as substituents for alkyl groups.

The term "effective amount" means the amount of the subject compound that will elicit the biological, physiological, pharmacological, therapeutic or medical response of a cell, tissue, system, body, animal, individual, patient or human that is being sought by the researcher, pharmacologist, pharmacist, veterinarian, medical doctor, or other clinician, e.g., lessening of the effects/symptoms of cell proliferative disorders such as a cancer or tumor, or killing or inhibiting growth of a proliferating cell, such as a tumor cell.

The term "further treated", "further administer" or "further administered", means that the different therapeutic agents may be administered together, alternatively or intermittently. Such further administration may be temporally or spatially separated, for example at different times, on different days or via different modes or routes of administration.

The term "halogen" or "halo" refers to fluorine, chlorine, bromine and iodine.

The term "IC50", as used herein, refers to concentrations at which a measurable phenotype or response, for example growth of cells such as tumor cells, is inhibited by 50%. IC50 values can be estimated from an appropriate dose-response curve, for example by eye or by using appropriate curve fitting or statistical software. More accurately, IC50 values may be determined using non-linear regression analysis.

As used herein, an "individual" means a multi-cellular organism, for example an animal such as a mammal, preferably a primate. In addition to primates, such as humans, a variety of other mammals can be treated according to the method described herein. For example, mammals including, but not limited to, cows, sheep, goats, horses, dogs, cats, guinea pigs, rats or other bovine, ovine, equine, canine, feline, rodent or murine species can be used.

The term "metabolite", as used herein, refers to any substance produced by metabolism or by a metabolic process. Metabolism, as used herein, refers to the various physical/chemical/biochemical/phamacological reactions involved in the transformation of molecules or chemical compounds occurring in the cell, tissue, system, body, animal, individual, patient or human therein.

The term "prodrug", as used herein, refers to an agent which is converted into a pharmacologically active parent drug in vivo. Prodrugs are often useful because, in some situations, they may be easier to administer than the parent drug. They may, for instance, be bioavailable by oral administration whereas the parent drug is not. The prodrug may also have improved solubility in pharmaceutical compositions over the parent drug. A prodrug may be converted into the parent drug by various mechanisms, including enzymatic processes and metabolic hydrolysis. See Gangwar et al., "Prodrug, molecular structure and percutaneous delivery", Des. Biopharm. Prop. Prodrugs Analogs, [Symp.] Meeting Date 1976, 409-21. (1977); Nathwani and Wood, "Penicillins: a current review of their clinical pharmacology and therapeutic use", Drugs 45(6): 866-94 (1993); Sinhababu and Thakker, "Prodrugs of anticancer agents", Adv. Drug Delivery Rev. 19(2): 241-273 (1996); Stella et al., "Prodrugs. Do they have advantages in clinical practice?", Drugs 29(5): 455-73 (1985); Tan et al. "Development and optimization of anti-HIV nucleoside analogs and prodrugs: A review of their cellular pharmacology, structure-activity relationships and pharmacokinetics", Adv. Drug Delivery Rev. 39(1-3): 117-151 (1999);

As used herein, a "proliferative disorder" includes a disease or disorder that affects a cellular growth, differentiation, or proliferation process. As used herein, a "cellular growth, differentiation or proliferation process" is a process by which a cell increases in number, size or content, by which a cell develops a specialized set of characteristics which differ from that of other cells, or by which a cell moves closer to or further from a particular location or stimulus. A cellular growth, differentiation, or proliferation process includes amino acid transport and degradation and other metabolic processes of a cell. A cellular proliferation disorder may be characterized by aberrantly regulated cellular growth, proliferation, differentiation, or migration. Cellular proliferation disorders include tumorigenic diseases or disorders. As used herein, a "tumorigenic disease or disorder" includes a disease or disorder characterized by aberrantly regulated cellular growth, proliferation, differentiation, adhesion, or migration, which may result in the production of or tendency to produce tumors. As used herein, a "tumor" includes a benign or malignant mass of tissue. Examples of cellular growth or proliferation disorders include, but are not limited to, cancer, e.g., carcinoma, sarcoma, or leukemia, examples of which include, but are not limited to, colon, ovarian, lungs breast, endometrial, uterine, hepatic, gastrointestinal, prostate, and brain cancer; tumorigenesis and metastasis; skeletal dysplasia; and hematopoietic and/or myeloproliferative disorders.

### III. Platinum-based compounds

According to the present invention, the platinum-based chemotherapeutic agent is satraplatin, or a metabolite of satraplatin. Satraplatin (JM216) has the structure:

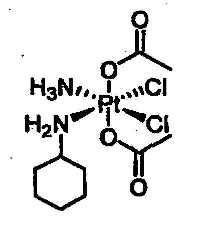

Satraplatin can be synthesised according to the method disclosed in U.S. Patent No. 5,072,011 and 5,244,919 or by appropriate modification of the method disclosed in US 6,518,428.

Upon administration of satraplatin to a cell, animal or a human patient, a number of related platinum-containing metabolites may be formed. The term "metabolite", as used herein, also includes a substance derived from a drug by physical, chemical, biological or biochemical processes in the body or cell after the drug is administered. Figure 1 (taken from Raynaud et al. 1996 Cancer Chemother Phamacol 38: 155-162) shows exemplary metabolites of satraplatin (JM216), and depicts JM118, JM383, JM518, JM559 and JM149. As will be appreciated by a person skilled in the art, additional platinum-containing molecules may be formed by metabolism of satraplatin after administration to a cell, animal or human patient, and such metabolites of satraplatin are encompassed in the scope of the instant invention. Suitable metabolites may be formed within the treated cell, animal or human by biological or biochemical biotransformation. Alternatively, such metabolites may be first formed out of the treated cell (such as in the GI tract), or may be formed by synthetic reaction from suitable starting materials and administered directly to the cell, animal or human patient. For example, JM118 may be synthesised according to the method disclosed in EP 147926, GB 2,060,615 and U.S. 4,329,299, or may be formed by biotransformation from JM216 in a separate fermentation step.

Satraplatin is considerably different from cisplatin due to its oral availability and favourable safety profile, such as the absence of kidney- and neurotoxicity. In addition, there is no cross resistance between satraplatin and cisplatin (Cancer Res, 53,2581; Br J Cancer 68, 240). Indeed, herein we demonstrate that the efficacy of satraplatin and its metabolite is maintained in cisplatin-resistant tumor cells (Example 4).

### IV. Non-platinum-based therapeutic agent

### 1. Taxanes

Resistance to taxanes like pacitaxel and docetaxol is a major problem for all chemotherapeutic regimens utilizing these drugs. Taxanes exert their cytotoxic effect by binding to tubulin, thereby causing the formation of unusually stable microtubules. The ensuing mitotic arrest triggers the mitotic spindle checkpoint and results in apoptosis. Other mechanisms that mediate apoptosis through pathways independent of microtubule dysfunction have been described as well, including molecular events triggered by the activation of Cell Division Control-2 (cdc-2) Kinase, phosphorylation of BCL-2 and the induction of interleukin 1β (IL-1β) and tumor necrosis factor-α (TNF-α). Furthermore, taxanes have been shown to also exert anti-tumor activity via mechanisms other than the direct activation of the apoptotic cascade. These mechanisms include decreased production of metalloproteinases and the inhibition of endothelial cell proliferation and motility, with consequent inhibition of angiogenesis.

As shown in the Examples, Applicants have demonstrated that exemplary subject platinum-based compounds, including satraplatin (JM216), maintain their therapeutic efficacy in tumor cell lines resistant to taxanes. In other words, tumor cell lines that are resistant to taxane treatment do not show resistance to treatment with the exemplary subject platinum-based compounds of the present invention. Thus, the present invention relates to the use of satraplatin (JM216) for treating patients with tumors resistant to taxanes.

By the term "taxane", it is meant to include any member of the family of terpenes, including, but not limited to paclitaxel (Taxol) and docetaxel (Taxotere), which were derived primarily from the Pacific yew tree, *Taxus_brevifolia*, and which have activity against certain tumors, particularly breast, lung and ovarian tumors (See, for example, Pazdur et al. Cancer Treat Res. 1993.19:3 5 1; Bissery et al. Cancer Res. 199151:4845). In the methods and packaged pharmaceuticals described herein, preferred taxanes are paclitaxel, docetaxel, deoxygenated paclitaxel, TL-139 and their derivatives. See Annu. Rev. Med. 48:353-374 (1997).

The term "taxane" as used herein includes both naturally derived and related forms and chemically synthesized terpenes or derivatives thereof, including deoxygenated paclitaxel compounds such as those described in U.S. Pat. Nos. 5,440,056 and 4,942,184 and that sold as TAXOL^{®} by Bristol-Myers Oncology. Pactitaxel has been approved for clinical use in the treatment of refractory ovarian cancer in the United States (Markman et al., Yale Journal of Biology and Medicine, 64:583, 1991; McGuire et al., Ann. Intern. Med., 111:273, 1989). It is effective for chemotherapy for several types of neoplasms including breast (Holmes et al., J. Nat. Cancer Inst., 83:1797,1991) and has been approved for treatment of breast cancer as well. It is a potential candidate for treatment of neoplasms in the skin (Einzig et al., Proc. Am. Soc. Clin. Oncol., 20:46) and head and neck carcinomas (Forastire et al. Sem. Oncol., 20:56,1990). The compound also shows potential for the treatment of polycystic kidney disease (Woo et al, Nature, 368:750, 1994), lung cancer and malaria. Docetaxel (N-debenzoyl-N-tert-butoxycarbonyl-10-descetyl paclitaxel) is produced under the trademark TAXOTERE® by Rhone-Poulenc Rorer S.A. In addition, other taxanes are described in "Synthesis and Anticancer Activity of Taxol other Derivatives," D. G. 1. Kingston et al., Studies in Organic Chemistry, vol. 26, entitled "New Trends in Natural Products Chemistry" (1986), Atta-urRabman, P. W. le Quesne, Eds. (Elvesier, Amsterdam 1986), pp 219-235 are incorporated herein. Various taxanes are also described in U.S. Patent No. 6,380,405, the entirety of which is incorporated herein.

The use described herein is applicable for treating tumors resistant to treatment by any taxane, regardless of the resistance mechanism. Known mechanisms that confer taxane resistance include, for example, molecular changes in the target molecules molecules, i.e., α-tublin and/or β-tubulin, up-regulation of P-glycoprotein (multidrug resistance gene MDR-1), changes in apoptotic regulatory and mitosis checkpoint proteins, changes in cell membranes, overexpression of interleukin 6 (IL-6; Clin Cancer Res (1999) 5, 3445-3453; Cytokine (2002) 17, 234-242), the overexpression of interleukin 8 (IL-8; Clin Cancer Res (1999) 5,3445-3453; Cancer Res (1996) 56, 1303-1308) or the overexpression of monocyte chemotactic protein-1 (MCP-1; (MCP-1; Clin Cancer Res (1999) 5, 3445-3453), changes in the levels of acidic and basic fibroblast growth factors, transmembrane factors, such as p185 (HER2; Oncogene (1996) 13, 1359-1365) or EGFR (Oncogene (2000) 19, 6550-6565; Bioessays (2000) 22, 673-680), changes in adhesion molecules, such as β1 integrin (Oncogene (2001) 20, 4995-5004), changes in house keeping molecules, such as glutathione-S-transferase ans/or glutathione peroxidase (Jpn J Clin Oncol (1996) 26, 1-5), changes in molecules involved in cell signalling, such as interferone response factor 9, molecules involved in NF-κB signalling, molecules involved in the PI-3 kinase/AKT survival pathway, RAP-1 kinase activity, PKC α/β or PKC β/β2 and via nuclear proteins, such as nuclear annexin IV, the methylation controlled J protein of the DNA J family of proteins, thymidylate synthetase or c-jun.

Another known mechanism that confers taxane resistance is, for example, changes in apoptotic regulatory and mitosis checkpoint proteins. Such changes in apoptotic regulatory and mitosis checkpoint proteins include the overexpresion of Bcl-2(Cancer Chemother Pharmacol (2000) 46, 329-337; Leukemia (1997) 11, 253-257) and the overexpresion of Bcl-xL (Cancer Res (1997) 57, 1109-1115; Leukemia (1997) 11, 253-257). Overexpresion of Bcl-2 may be effected by estradiol (Breast Cancer Res Treat (1999) 42,73-81).

Taxane resistance may also be conferred via changes in the cell membrane. Such changes include the change of the ratio of fatty acid methylene:methyl (CancerRes (1996) 56, 3461-3467), the change of the ratio of choline:methyl(CancerRes (1996)56, 3461-3467) and a change of the permeability of the cell membrane (J Cell Biol (1986) 102, 1522-1531).

Further known mechanisms that confer taxane resistance are changes in acidic and basic fibroblast growth factors (Proc Natl Acad Sci USA (2000) 97, 8658-8663), molecule involved in cell signalling, such as interferone response factor 9 (Cancer Res (2001) 61, 6540-6547), molecules involved in NF-κB signalling (Surgery (1991) 130, 143-150), molecules involved in the PI-3 kinase/AKT survival pathway (Oncogene (2001) 20, 4995-5004), RAF-1 kinase activity (Anticancer Drugs (2000) 11, 439-443; Chemotherapy (2000) 46, 327-334), PKC α/β (Int J Cancer (1993) 54" 302-308) or PKC β/β2 (Int J Cancer (2001) 93, 179-184, Anticancer Drugs (1997) 8, 189-198).

Taxane resistance may also be conferred via changes nuclear proteins, such as nuclear annexin IV (Br J Cancer (2000) 83, 83-88), the methylation controlled J protein of the DNA J family of proteins (Cancer Res (2001) 61, 4258-4265), thymidylate synthetase (Anticancer Drugs (1997) 8, 189-198) or c-jun (Anticancer Drugs (1997) 8, 189-198), via paracrine factors, such as LPS (J Leukoc Biol (1996) 59,280-286), HIF-1 (Mech Dev (1998) 73, 117-123), VEGF (Mech Dev (1998) 73, 117-123) and the lack of decline in bcl-XL in spheroid cultures (Cancer Res (1997) 57, 2388-2393).

Applicants have demonstrated that the subject platinum-based agents are effective in treating resistant tumors in which resistance is mediated through at least one of the following three mechanisms: multidrug resistance, tubulins and topoisomerase I. This section describes these three resistance mechanisms and therapeutic agents for which resistance arises through at least one of these mechanisms. One of skill in the art will understand that tumor cells may be resistant to a chemotherapeutic agent through more than one mechanism. The resistance of tumor cells to paclitaxel may be mediated through via multidrug resistance, or alternatively or additionally, via tubulin mutation(s).

### a Resistance mediated through tubulins

Microtubules are intracellular filamentous structures present in all eukaryotic cells. As components of different organelles such as mitotic spindles, centrioles, basal bodies, cilia, flagella, axopodia and the cytoskeleton, microtubules are involved in many cellular functions including chromosome movement during mitosis, cell motility, organelle transport, cytokinesis, cell plate formation, maintenance of cell shape and orientation of cell microfibril deposition in developing plant cell walls. The major component of microtubules is tubulin, a protein composed of two subunits called alpha and beta. An important property of tubulin in cells is the ability to undergo polymerization to form microtubules or to depolymerize under appropriate conditions. This process can also occur in vitro using isolated tubulin.

Microtubules play a critical role in cell division as components of the mitotic spindle, an organelle which is involved in distributing chromosomes within the dividing cell precisely between the two daughter nuclei. Various drugs prevent cell division by binding to tubulin or to microtubules. Anticancer drugs acting by this mechanism include the alkaloids vincristine and vinblastine, and the taxane-based compounds paclitaxel and docetaxel {see, for example, E. K. Rowinsky and R. C. Donehower, Pharmacology and Therapeutics, 52, 35-84 (1991)}. Other antitubulin compounds active against mammalian cells include benzimidazoles such as nocodazole and natural products such as colchicine, podophyllotoxin, epithilones, and the combretastatins.

Non-platinum-based therapeutic agents may exert their activities by, for example, binding to α-tubulin, β-tubulin or both, and/or stabilizing microtubules by preventing their depolymerization. Other modes of activity may include, down regulation of the expression of such tubulin proteins, or binding to and modification of the activity of other proteins involved in the control of expression, activity or function of tubulin.

In one embodiment, the resistance of tumor cells to a taxane agent is mediated through tubulin. By "mediated through tubulin", it is meant to include direct and indirect involvement of tubulin. For example, resistance may arise due to tubulin mutation, a direct involvement of tubulin in the resistance. Alternatively, resistance may arise due to alterations elsewhere in the cell that affect tubulin and/or microtubules. These alterations may be mutations in genes affecting the expression level or pattern of tubulin, or mutations in genes affecting microtubule assembly in general. Mammals express 6 α- and 6 β-tubulin genes, each of which may mediate drug resistance.

Specifically, tubulm-mediated tumor resistance to non-platinum-based therapeutic agents may be conferred via molecular changes in the tubulin molecules. For example, molecular changes include mutations, such as point mutations, deletions or insertions, splice variants or other changes at the gene, message or protein level. In particular embodiments, such molecular changes may reside in amino acids 250-300 of β-tubulin, or may effect nucleotides 810 and/or 1092 of the β-tubulin gene. For example, and without wishing to be limited, the paclitaxel-resistant human ovarian carcinoma cell line 1A9-PTX10 is mutated at amino acid residues β 270 and β 364 of β-tubulin (see Giannakakou et al., 1997). For another example, two epothilone-resistant human cancer cell lines has acquired β-tubulin mutations at amino acid residues β274 and β282, respectively (See Giannakakou et al., 2000). These mutations are thought to affect the binding of the drugs to tubulins. Alternatively, mutations in tubulins that confer drug resistance may also be alterations that affect microtubule assembly. This change in microtubule assembly has been demonstrated to compensate for the effect of drugs by having diminished microtubule assembly compared to wild-type controls (Minotti, A. M., Barlow, S. B., and Cabral, F. (1991) J Biol Chem 266, 3987-3994). It will also be understood by a person skilled in the art that molecular changes in α-tubulin may also confer resistance to certain compounds. WO 00/71752 describes a wide range of molecular changes to tubulin molecules and the resistance to certain chemotherapeutic compounds that such molecular changes may confer on a cell.

Tubulin-mediated tumor resistance to non-platinum-based therapeutic agents may also be conferred via alterations of the expression pattern of either α-tubulin or the β-tubulin, or both. For example, several laboratories have provided evidence that changes in the expression of specific β-tubulin genes are associated with paclitaxel resistance in cultured tumor cell lines (Haber, M., Burkhart, C. A., Regl, D. L., Madafiglio, J., Norris, M. D., and Horwitz, S. B. (1995) J BioL Chem. 270, 31269-75; Jaffrezou, J. P., Dumontet, C., Deny, W. B., Duran, G., Chen, G., Tsuchiya, E., Wilson, L., Jordan, M. A., and Sikic, B.1. (1995) Oncology Res. 7,517-27; Kavallaris, M., Kuo, D. Y. S., Burkhart, C. A., RegI, D. L., Norris, M.D., Haber, M., and Horwitz, S. B. (1997) J Clin.Invest. 100, 1282-93; and Ranganathan, S., Dexter, D. W., Benetatos, C. A., and Hudes, G. R. (1998) Biochi,. Biophys. Acta 1395, 237-245).

Tubulin-mediated tumor resistance to non-platinum-based therapeutic agents may also be conferred via an increase of the total tubulin content of the cell, an increase in the α-tubulin content or the expression of different electrophoretic variants of α-tubulin. Furthermore, resistance may be conferred via alterations in the electrophoretic mobility of β-tubulin subunits, overexpression of the Hβ2 tubulin gene, overexpression of the Hβ3 tubulin gene, overexpression of the Hβ4 tubulin gene, overexpression of the Hβ4a tubulin gene or overexpression of the Hβ5 tubulin gene,

Tubulin-mediated tumor resistance to non-platinum-based therapeutic agents may also be conferred via post-translational modification of tubulin, such as increased acetylation of α-tubulin (Jpn J Cancer Res (85) 290-297), via proteins that regulate microtubule dynamics by interacting with tubulin dimmers or polymerized microtubules. Such proteins include but are not limited to stathmin (Mol Cell Biol (1999) 19, 2242-2250) and MAP4 (Biochem Pharmacol (2001) 62, 1469-1480).

Chemotherapeutic agents for which resistance is at least partly mediated through tubulin include, taxanes (paclitaxel, docetaxel and derivatives thereof), vinca alkaloids (vinblastine, vincristine, vindesine and vinorelbine), epothilones (epothilone A, epothilone B and discodermolide), nocodazole, colchicine, colchicine derivatives, allocolchicine, Halichondrin B, dolstatin 10, maytansine, rhizoxin, thiocolchicine, trityl cysterin, estramustine and nocodazole. See WO 03/099210 and Giannakakou et al., 2000. Additional chemotherapeutic agents for which resistance is at least partly mediated through tubulin include, colchicine, curacin, combretastatins, cryptophycins, dolastatin, auristatin PHE, symplostatin 1, eleutherobin, halichondrin B, halimide, hemiasterlins, laulimalide, maytansinoids, PC-SPES, peloruside A, resveratrol, S-allylmercaptocysteine (SAMC), spongistatins, taxanes, vitilevuamide, 2-methoxyestradiol (2-ME2), A-289099, A-293620/A-318315, ABT-751/E7010, ANG 600 series, anhydrovinblastine (AVLB), AVE806, bivatuzumab mertansine, BMS-247550, BMS-310705, cantuzumab mertansine, combretastatin, combretastatin A-4 prodrug (CA4P), CP248/CP461, D-24851/D-64131, dolastatin 10, E7389,BP0906, FR182877, HMN-214, huN901-DM1/BB-10901TAP, ILX-651, KOS-862, LY355703, mebendazole, MLN591DM1, My9-6-DM1, NPI-2352 and NPI-2358, Oxi-4503, R440, SB-715992, SDX-103, T67/T607, trastuzumab-DM1, TZT-1027, vinflunine, ZD6126, ZK-EPO.

Chemotherapeutic agents for which resistance is at least partly mediated through tubulin are taxanes, including, but not limited to paclitaxel and docetaxel (Taxotere), which were derived primarily from the Pacific yew tree, Taxus brevifolia, and which have activity against certain tumors, particularly breast and ovarian tumors (See, for example, Pazdur et al. Cancer Treat Res.1993.19:3 5 1; Bissery et al. Cancer Res. 1991 51:4845).

### b. Resistance mediated through multidrug resistance

In another embodiment, the resistance of tumor cells to a non-platinum-based therapeutic agent is mediated through multidrug resistance. The term "multidrug resistance (MDR)", as used herein, refers to a specific mechanism that limits the ability of a broad class of hydrophobic, weakly cationic compounds to accumulate in the cell. These compounds have diverse structures and mechanisms of action, yet all are affected by this mechanism.

Experimental models demonstrate that multidrug resistance can be caused by increased expression of ATP-binding cassesette (ABC) transporters, which function as ATP-dependent efflux pumps. These pumps actively transport a wide array of anti-cancer and cytotoxic drugs out of the cell, in particular natural hydrophobic drugs. In mammals, the superfamily of ABC transporters includes P-glycoprotein (P-gp, ABCB1) transporters (MDR1 and MDR3 genes in human), the MRP subfamily (already composed ofsix members, e.g MRP1 (ABCC1)), and bile salt export protein (ABCB11; Cancer Res (1998) 58, 4160-4167), MDR-3 (Nature Rev Cancer (2002) 2,48-58), lung resistance protein (LRP) and breast cancer resistant protein (BCRP, ABCG2). See Kondratov et al., 2001 and references therein; Cancer Res (1993) 53, 747-754; J Biol Chem (1995) 270, 31269-31275; Leukemia (1994) 8, 465-475; Biochem Pharmacol (1997) 53,461-470). These proteins can recognize and efflux numerous substrates with diverged chemical structure, including many anticancer drugs. Overexpression of P-gp is the most common cause for MDR. Other causes of MDR have been attributed to changes in topoisomerase II, protein kinase C and specific glutathione transferase enzymes. See Endicott and Ling, 1989.

The uses described herein are useful for treating tumors resistant to a taxane, in which resistance is at least partially due to MDR. In a preferred embodiment, the drug resistance of the tumor is mediated through overexpression of an ABC transporter. In a further preferred embodiment, the drug resistance of the tumor is mediated through the overexprssion of P-gp. Numerous mechanisms can lead to overexpression of P-gp, including amplification of the MDR-1 gene (Anticancer Res (2002) 22, 2199-2203), increased transcription of the MDR-1 gene (J Clin Invest (1995) 95, 2205-2214; Cancer Lett (1999) 146, 195-199; Clin Cancer Res (1999) 5, 3445-3453; Anticancer Res (2002) 22, 2199-2203), which may be mediated by transcription factors such as RGP8.5 (Nat Genet 2001 (27), 23-29), mechanisms involving changes in MDR-1 translational efficiency (Anticancer Res (2002) 22, 2199-2203), mutations in the MDR-1 gene (Cell (1988) 53, 519-529; Proc Natl Acad Sci USA (1991) 88, 7289-7293; Proc Natl Acad Sci USA (1992) 89, 4564-4568) and chromosomal rearrangements involving the MDR-1 gene and resulting in the formation of hybrid genes (J Clin Invest (1997) 99, 1947-1957).

Therapeutic agents to which resistance is conferred via the action of P-gp include, but is not limited to: vinca alkaloids (e.g., vinblastine), the anthracyclines (e.g., adriamycin, doxorubicin), the epipodophyllotoxins (e.g., etoposide), taxanes (e.g., paclitaxel, docetaxel), antibiotics (e.g., actinomycin D and gramicidin D), antimicrotubule drugs (e.g., colchicine), protein synthesis inhibitor (e.g., puromycin), toxic peptides (e.g., valinomycin), topoisomerase inhibitors (e.g., topotecan), DNA intercalators (e.g., ethidium bromide) and anti-mitotics. See WO 99/20791.

### c. Resistance mediated through toposiomerase I

### V. Assays for effectiveness of treatment

In one embodiment, the platinum-based compounds described herein kill tumor cells resistant to a non-platinum-based therapeutic agent. Viability of a tumor cell can be determined by any methods known in the art. For example, one may use the colorimetric cytotoxicity assay described for anticancer drug screening in Shekan et al., J. Natl. Cancer, Inst. 82:1107-12 (1990). For another example, one may determine the viability of a tumor cell by contacting the cell with a dye and viewing it under a microscope. Viable cells can be observed to have an intact membrane and do not stain, whereas dying or dead cells having "leaky" membranes do stain. Incorporation of the dye by the cell indicates the death of the cell. A dye useful for this purpose is trypan blue.

The exemplary platinum-containing composition prepared according to the use of the present invention may induce apoptosis, a mode of cell death, in resistant tumor cells. Apoptosis is recognized by a characteristic pattern of morphological, biochemical and molecular changes. Cells going through apoptosis appear shrunken and rounded. They also can be observed to become detached from a culture dish in which they are maintained. The morphological changes involve a characteristic pattern of condensation of chromatin and cytoplasm which can be readily identified by microscopy. When stained with a DNA-binding dye, e.g., H33258, apoptotic cells display classic condensed and punctuate nuclei instead of homogenous and round nuclei.

A typical characteristic of apoptosis is endonucleolysis, a molecular change in which nuclear DNA is initially degraded at the linker sections of nucleosomes to give rise to fragments equivalent to single and multiple nucleosomes. When these DNA fragments are subjected to gel electrophoresis, they reveal a series of DNA bands which are positioned approximately equally distant from each other on the gel. The size difference between the two bands next to each other is about the length of one nucleosome, i.e., 120 base pairs. This characteristic display of the DNA bands is called a DNA ladder and it indicates apoptosis of the cell. Apoptotic cells can also be identified by flow cytometric methods based on measurement of cellular DNA content, increased sensitivity of DNA to denaturation, or altered light scattering properties. These methods are well known in the art. It should be recognized however, that modes of programmed cell death, including apoptosis, may following a number of mechanisms or show other phenotypes/properties to those described above. In such cases, these mechanisms may also be characterized, classified or considered as "apoptosis".

Cytotoxicity may also be measured using the SRB assay according to Shekan et al (J Natl Cancer Inst (1990) 82, 1107-112), as described in the Examples.

Additional assays for cell viability are described in Chapter 15 of Handbook of Fluorescent Probes and Research Products (Molecular Probes Handbook), which is incorporated in its entirety herein.

In another embodiment, the platinum-based compounds described herein inhibit the growth of tumor cells resistant to a non-platinum-based therapeutic agent. The growth inhibition of resistant tumor cells caused by a platinum-based compound can be either partial (slowing down cell growth) or complete inhibition (i.e., arresting cells at a certain point in cell cycle). Cell growth can be measured by any techniques known in the art. Such techniques include, for example, MTT assay (based on reduction of the tetrazolium salt 3, [4,5-dimethylthiazol-2-yl]-2,5-diphenytetrazolium bromide), and PicoGreen assay using the DNA-binding dye Picogreen, both of which are described in Torrance, et al., Nat. Biotech.19:940-945 (2001). Other assays for cell proliferation/growth are described in Chapter 15 of Handbook of Fluorescent Probes and Research Products (Molecular Probes Handbook).

Progression of disease, cancer or tumor in response to treatment using the subject platinum-based compounds can be monitored using any standard technique known in the art. For example, tumor size can be monitored and assessed to see if tumor size reduction has occurred as a result of the treatment. Monitoring and assessment may be aided by a variety of means including biopsies, manual inspection, microscopy, whole or partial body imaging and scans, and various molecular-based diagnostic and prognostic methods including those that investigate tumor-specific markers or mutations.

### VI. Tumors and Other Proliferative Disorders

The subject platinum-based compound is useful to treat proliferative disorders resistant to a non-platinum-based therapeutic agent. The term "proliferative disorder" is also art-recognized and further includes a disorder affecting an animal in a manner which is marked by aberrant, or otherwise unwanted, proliferation of a subset of cells of an animal. Cancer and tumors are proliferative disorders. Cells comprising or derived from a tumor will generally be understood to be a proliferating cell, typically a hyper-proliferating cell, and in other circumstances, a tumor cell may be dysplastic, or may have proliferated.

Tumors that are resistant or refractory to treatment with a variety of chemotherapeutic agents may benefit from treatment with the use described herein. Suitable tumors may be solid tumors, which are cancer of body tissues other than blood, bone marrow, or the lymphatic system. Preferred tumors are those resistant to non-platinum-based chemotherapeutic agents. Suitable tumors may also be hematological tumors, such as leukemia and lymphomas. Leukemia is a collective term for malignant diseases characterized by a proliferation of malignantly changed white blood cells. Diseases arising from lymphatic tissue are called lymphomas.

Solid tumors may be selected from: liver cancer, stomach cancer, colon cancer, breast cancer, pancreas cancer, prostate cancer, skin cancer, renal cancer, bone cancer, skin cancer, cervical cancer, ovarian cancer, lung cancer, bronchial, small and non-small-cell lung cancer, gastric, prostate, pancreas, and head and neck cancer.

Hematological tumors may be leukemia, such as Acute Myelogenous Leukemia (AML), Acute Lymphoblastic Leukemia (ALL), Acute Leukemia, Acute Promyelocytic Leukemia, Chronic Granulocytic Leukemia (CGL), Chronic Leukemia, Chronic Lymphocytic Leukemia (CLL), Chronic Myelogenous Leukemia (CML), Chronic Myelomonocytic Leukemia, Common-type Acute Lymphoblastic Leukemia, Eosinophilic Leukemia, Erythroleukemia, Extranodal Lymphoma, Follicular Lymphoma, Hairy Cell Leukemia, Monocytic Leukemia, and Prolymphocytic Leukemia.

Hematological tumors may also be lymphoma, such as B Cell Lymphomas, Burkitt Lymphoma, Cutaneous T Cell Lymphoma, High-Grade Lymphoma, Hodgkin Lymphoma, Non-Hodgkin Lymphoma, Low-grade Lymphoma, Lymphoblastic Lymphoma, Mantle Cell Lymphoma, Marginal Zone Lymphoma, Mucosa-Associated Lymphoid Tissue (MALT) Lymphomas, T Cell Lymphomas, peripheral T cell lymphoma, multiple myeloma, Essential Thrombocythemia, Extramedullary myeloma, and Granulocytic Sarcomae.

Tumors resistant or refractory to taxanes include, for example, breast cancer, cervical cancer, colorectal cancer, peritoneal cancer, ovarian cancer, bronchial cancer, small cell lung cancer, non-small cell lung cancer, gastric, prostate, and head and neck cancer, including recurrent squamous cell carcinomas.

The term "resistant", as used herein, include both partially resistant and completely resistant. Thus, a tumor that is only partially resistant to a taxane may nonetheless benefit from treatment with the subject platinum-based compound. Indeed, in certain embodiments it may be beneficial to treat a tumor if such resistance is merely suspected, may not yet be know or even before such resistance has developed. In such cases, a co-administration, combination-thereapy or treatment regime may be envisioned, by appropriate use of the subject platinum-based compounds together with the appropriate non-platinum-based therapeutic agent. In alternative embodiments of all aspects of the instant invention, the subject platinum-based compounds will be useful in treating individuals suffering from a cancer or a tumor that has been previously treated with a taxane. In such embodiments, it may be subsequently determined, or not at all, that the cancer or tumor was resistant or refractory to the non-platinum-based therapeutic agent.

Cell lines that may be used to evaluate whether the compounds described herein possess cytotoxic activity against drug-resistant cell lines include but are not limited to the taxene-resistant tubulin-mutated cell lines 1A9-PTX10 and 1A9-PTX22 and their parental cell line 1A9 (J Biol Chem (1997) 272, 17118-17124), the adriamycin-resistact P-gp overexpressing cell line NCI-Adr resistant (Vickers et al., 1989. Mol Endocrinology 3 (1):157-164), the camptothecin-resistant cell lines CEM/C1 and CEM/C2 and their parental cell line CBM (Kapoor et al., 1995. Oncology Research 7; 83-95), the TWIST overexpressing cell line HNE1-T3 and its parental cell line (Oncogene (2004) 23, 474-482), the paclitaxel-resistant of the human ovarian cancer cell line SKOV-3 and SKOV-3 (Clin Cancer Res (2003) 9, 2778-2785), the mitoxantrone-resistant colon cancer carcinome cell line HT29/MIT and its parental cell line HT29 (Cancer Res (2001) 61, 6034-6037), and the etoposide-resistant breast cancer cell line MCF-7/VP and its parental cell line MCF-7 (Cancer Res (1994) 54, 152-158; Int J Cancer (1997) 71, 35-41).

The subject platinum-based compound is also believed useful in treating other types of proliferative disorders, including, proliferative disorders which are characterized by benign indications. Such disorders may also be known as "cytoproliferative" or "hyperproliferative" in that cells are made by the body at an atypically elevated rate. Such disorders include, but are not limited to, the following: hemangiomatosis in new born, secondary progressive multiple sclerosis, chronic progressive myelodegenerative disease, neurofibromatosis, ganglioneuromatosis, keloid formation, Paget's disease of the bone, fibrocystic disease of the breast, Peronies and Duputren's fibrosis, restenosis and cirrhosis.

### VII. Combination Therapy

The pharmaceutical compositions prepared according to the claimed use can be co-administered, e.g., in the same or different formulation, with a variety of other drugs. For example, the pharmaceutical compositions can be used as part of a regiment of treatment in which they are combined with other chemotherapeutic agents including anti-cancer therapeutic agents that inhibit cancer growth, anti-angiogenesis agents and anti-metastatic agents. The pharmaceutical compositions may also be combined with immunomodulators.

Preferably, the pharmaceutical compositions are co-administered with an agent that tackles and/or overcomes a specific drug resistance mechanism. In this context, a "specific drug resistance mechanism" refers to any physiological or cellular mechanism which causes a cancer, tumor, cancer cell or tumor cell to become resistant to an anti-cancer therapeutic agent, but which drug resistance mechanism can be overcome, i.e. resistance is circumvented, by administering suitable compounds, agents or pharmaceutical agents. For example, when a tumor drug resistance is caused by an increase in drug efflux brought about by an overexpression of ATP-dependent pumps such as P-glycoprotein, pharmacological agents that reverse the excessive drug efflux through P-glycoprotein can be used in combination with the subject pharmaceutical compositions for treating resistance tumors. Therefore, the pharmaceutical compositions can be co-administered with an agent that overcomes a specific drug resistance mechanism. More preferably, said specific drug resistance mechanism is an increase in drug efflux brought about by ATP-binding cassette transporters. Such suitable pharmacological agents include, for example, phenothiazines, verapamil, tamoxifen, quinidine, phenothiazines, cyclosporine A, methylenedioxymethamphetamine, methylenedioxyethylamphetamine, paramethoxyamphetamine, pervilleine F, PSC 833 and LY335979, among others. For a general discussion of the pharmacologic implications for the clinical use of P-glycoprotein inhibitors, see Lum et al., Drug Resist. Clin. Onc. Hemat., 9: 319-336 (1995); Schinkel et al., Eur. J. Cancer, 31A: 1295-1298 (1995).

One particular drug that can be combined with the subject pharmaceutical compositions is PSC-833 (Valspodar), an analogue of cyclosporine. PSC-833 was found to be a P-gp inhibitor 10 times more potent than cyclosporine, and without its side-effect of nephrotoxicity and immunosuppression. Although combination phase I and II studies in different tumor types showed that PSC-833 had a profound effect on the pharmacokinetics of the co-administered chemotherapy, this effect could be adjusted for by reducing the dose of chemotherapy given. See Baird and Kaye, 2003.

In another embodiment, when a tumor drug resistance is caused by a mutation in β-tubulin, cancer therapeutic agents that have cellular targets other than microtubules may be used in combination with the pharmaceutical compositions.

In a further embodiment, the subject platinum-based compound is administered to a patient to whom an anti-emetic agent is also administered. Anti-emetic agents include any anti-emetic agents known to the skill artisan, including, but not limited to, serotonin-3 receptor antagonists like granisetron, ondansetron and tropisetron, NK1 receptor antagonists, antihistamines such as cinnarizine, cyclizine and promethazine, histamine H2 receptor antagonists such as ranitidine (Zantac), phenothiazines such as chlorpromazine, droperidol, haloperidol, methotrimeprazine, perphenazine, trifluoperazine and prochlorperazine, domperidone, and metoclopramide.

In other embodiments, the subject platinum-based compound is administered to a patient who is also treated with an anti-diarrheal such as loperamid, corticosteroide such as cortisone, growth hormone or growth factor such as GCSF or erythropoietin, a diuretica such as furosemid, steroidal or non-steroidal analgesics such as an opiate, e.g. morphine, or paracetamol or anti-hyperuricemics such as allopurinol.

In other embodiments, the subject platinum-based compound is administered to a patient, who is also treated with thrombocytes, erythrocytes or whole blood.

In other embodiments, the subject platinum-based compound is administered to a patient, who is also treated with stem cells of the bone marrow.

The term "co-administer" or "co-administered", as used herein, include administering two or more different therapeutic agents concurrently, sequentially or intermittently in all of the various aspects described herein or of the use of the invention. Thus, the subject platinum-based compounds may be administered before, after, or together with one or more other therapeutic agents to an individual in need of. In one embodiment, two or more therapeutic agents are formulated together with the subject platinum-based compound in a single pill.

The use described herein or the use according to the present invention can also be combined with other cancer treatments, such as radiation therapy, surgery, immunotherapy.

### VIII. Pharmaceutical formulations

The compositions described herein can be formulated and administered to treat individuals with cancer resistant to a non-platinum-based therapeutic agent by any means that produces contact of the active ingredient with the agent's site of action in the body of said individual, e.g. a mammal. They can be administered by any conventional means available for use in conjunction with pharmaceuticals, either as individual therapeutic active ingredients or in a combination of therapeutic active ingredients, such as by further administering another different therapeutic agent. They can be administered alone, but are generally administered with a pharmaceutical carrier selected on the basis of the chosen route of administration and standard pharmaceutical practice.

Pharmaceutical compositions for use in accordance with the present invention may be formulated in conventional manner using one or more physiologically acceptable carriers or excipients. The pharmaceutical compositions described herein can be formulated for a variety of routes of administration including systemic and topical or localized administration. Techniques and formulations generally may be found in Remmington's Pharmaceutical Sciences, Meade Publishing Co., Easton, PA. For systemic administration, injection is preferred, including intramuscular, intravenous, intraperitoneal, and subcutaneous (i.m., i.v., i.p., and i.c. respectively). For injection, the pharmaceutical compositions described herein can be formulated in liquid solutions, preferably in physiologically compatible buffers such as Hank's solution or Ringer's solution. In addition, the pharmaceutical compositions may be formulated in solid form and redissolved or suspended immediately prior to use. Lyophilized forms are also included.

The most preferred administration route is oral. In oral administration, the pharmaceutical compositions may take the form of, for example, unit dose-forms such as tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, cellulose derivatives or hydrogenated edible fats); emulsifying agents (e.g., lecithin or acacia); non-aqueous vehicles (e.g., almond oil, oily esters, ethyl alcohol or fractionated vegetable oils); and preservatives (e.g., methyl or propyl-p-hydroxybenzoates or sorbic acid). The preparations may also contain buffer salts, flavoring, coloring and sweetening agents as appropriate.

Preparations for oral administration may be suitably formulated to give controlled release of the active agent. For buccal administration the therapeutic compositions may take the form of tablets or lozenges formulated in a conventional manner. For administration by inhalation, the compositions for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the therapeutic agents and a suitable powder base such as lactose or starch.

The pharmaceutical compositions prepared according to the claimed use may be formulated for parenteral administration by injection, e.g., by bolus injection or continuous infusion. Formulations for injection may be presented in unit dosage form, e.g., in ampoules or in multi-dose containers, with an added preservative. The compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

In addition to the formulations described previously, the pharmaceutical compositions may also be formulated as a depot preparation. Such long acting formulations may be administered by implantation (for example subcutaneously or intramuscularly) or by intramuscular injection. Thus, for example, the therapeutic compositions may be formulated with suitable polymeric or hydrophobic materials (for example as an emulsion in an acceptable oil) or ion exchange resins, or as sparingly soluble derivatives, for example, as a sparingly soluble salt.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the compositions described herein are formulated into ointments, salves, gels, or creams as generally known in the art. A wash solution can be used locally to treat an injury or inflammation to accelerate healing. For oral administration, the therapeutic compositions are formulated into conventional oral administration forms such as capsules, tablets, and tonics.

The pharmaceutical compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. In other embodiments, the pack or dispenser may be further packaged in an outer carton.

A pharmaceutical composition can also be formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566. The pharmaceutical compositions can be used to provide slow or sustained release of one or more of the active ingredients using, for example, hydropropylmethyl cellulose, other polymer matrices, gels, permeable membranes, osmotic systems, multilayer coatings, microparticles, liposomes, microspheres, or the like, or a combination thereof to provide the desired release profile in varying proportions. Suitable sustained release formulations know to those of ordinary skill in the art, including those described herein, may be readily selected for use with the pharmaceutical compositions. Thus, single unit dosage forms suitable for oral administration, such as, but not limited to, tablets, capsules, gelcaps, caplets, powders, and the like, that are adapted for sustained release are suitable.

The pharmaceutical compositions may be formulated in a neutral or salt form. Pharmaceutical-acceptable salts include the acid addition salts and are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

### Dosage

The dosage administered will be a therapeutically effective amount of the compound sufficient to result in amelioration of symptoms of the cancer or tumor and will, of course, vary depending upon known factors such as the pharmacodynamic characteristics of the particular active ingredient and its mode and route of administration; age, sex, health and weight of the recipient; nature and extent of symptoms; kind of concurrent treatment, frequency of treatment and the effect desired.

Toxicity and therapeutic efficacy of pharmaceutical compositions described herein can be determined by standard pharmaceutical procedures in cell cultures or experimental animals, e.g., for determining the LD50 (the dose lethal to 50% of the population) and the ED50 (the dose therapeutically effective in 50% of the population). The dose ratio between toxic and therapeutic effects is the therapeutic index and it can be expressed as the ratio LD50/ED50. Therapeutic agents which exhibit large therapeutic indices are preferred. While therapeutic compositions that exhibit toxic side effects may be used, care should be taken to design a delivery system that targets such therapeutic agents to the site of affected tissue in order to minimize potential damage to uninfected cells and, thereby, reduce side effects.

The data obtained from cell culture assays and animal studies can be used in formulating a range of dosage for use in humans. The dosage lies preferably within a range of circulating concentrations that include tha ED50 with little or no toxicity. The dosage may vary within this range depending upon the dosage form employed and the route of administration utilized. For any agents used in the method described herein, the therapeutically effective dose can be estimated initially from cell culture assays. A dose may be formulated in animal models to achieve a circulating plasma concentration range that includes the IC50 (i.e., the concentration of the test therapeutic agent which achieves a half-maximal inhibition of symptoms or inhibition of biochemical activity) as determined in cell culture. Such information can be used to more accurately determine useful doses in humans. Levels in plasma may be measured, for example, by high performance liquid chromatography.

It is understood that appropriate doses of therapeutic agents depend upon a number of factors known to those or ordinary skill in the art, e.g., a physician. The dose(s) of the small molecule will vary, for example, depending upon the identity, size, and condition of the subject or sample being treated, further depending upon the route by which the composition is to be administered, if applicable, and the effect which the practitioner desires the therapeutic to have upon the therapeutic target or targets, such as the nucleic acid or polypeptide, through which the disease causes, symptoms or effects are mediated.

Exemplary doses include milligram or microgram amounts of the small molecule per kilogram of subject or sample weight, e.g., about 1 microgram per kilogram to about 500 milligrams per kilogram, about 100 micrograms per kilogram to about 50 milligrams per kilogram, or about 1 milligram per kilogram to about 5 milligrams per kilogram. A person skilled in the art will appreciate that doses can also be calculated on a body surface basis. A person of 70 kg has an approximate body surface area of 1.8 square meterdoses include milligram or microgram amounts of the small molecule per body surface area of subject or sample, e.g. about 50 microgram per square meter to about 15 grams per square meter, about 5 milligrams per square meter to about 1.5 grams per square meter, or about 50 milligram per square meter to about 150 milligrams per square meter.

The practice of aspects of the present invention may employ, unless otherwise indicated, conventional techniques of cell biology, cell culture, molecular biology, transgenic biology, microbiology, recombinant DNA, and immunology, which are within the skill of the art. Such techniques are explained fully in the literature. See, for example, Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Patent No: 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the treatise, Methods In Enzymology (Academic Press, Inc., N.Y.); Gene Transfer Vectors For Mammalian Cells (J. H. Miller and M. P. Calos eds., 1987, Cold Spring Harbor Laboratory); Methods In Enzymology, Vols. 154 and 155 (Wu et al. eds.), Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986); Manipulating the Mouse Embryo, (Cold Spring Harbor Laboratory Press, Cold Spring Harbor, N.Y., 1986).

### EXEMPLIFICATION

### EXAMPLE 1. Efficacy of satraplatin and its metabolites is maintained in taxane-resistant tumor cells

### A. Taxane-resistant tumor cells in which resistance is mediated through P-glycoprotein

We observed the surprising finding that subject platinum-based compounds described herein were useful in inhibiting or killing tumour cells that were resistant to other chemotherapeutic agents, where such resistance is mediated through p-glycoprotein.

Cells expressing P-glycoprotein (NCI-Adr resistant subline) were highly resistant to Adriamycin in the absence of verapamil but remained susceptible in the presence of verapamil, an inhibitor of P-glycoprotein (relative resistance was 115-fold), Upon treatment with JM216 and JM118 no change in the relative resistance was observed (relative resistance in individual experiments ranged between 1.0 to 1.5-fold).

The P-glycoprotein expressing NCI-Adr resistant subline (Vickers et al., 1989. Mol Endocrinology 3 (1):157-164) was used. 3,000-15,000 cells/well were exposed to the test compounds for 48 hours at various concentrations in the presence and absence of verapamil at 12.5 µg/ml in order to calculate the IC50 values shown in Tables 1, and cytotoxicty was measured using the SRB assay according to Shekan et al (J Natl Cancer Inst (1990) 82,1107-112). Briefly, cells were plated in 96 well dishes 24 hours prior to compound addition. Where noted, they were exposed to verapamil for 4 hours prior to compound addition and then throughout the treatment period (48 hours). The assay was terminated with the addition of cold TCA to a final concentration of 10% and the plates were incubated for one hour at 4 °C. The plates were then washed 5 times with water and 100 µl of a Sulforhodamine B solution (4%) was added to each well. The plate was then incubated for 10 minutes at room temperature before removal of unbound dye by washing with 1% acetic acid. The bound dye was solubilized with 10 mM Trizma base and the absorbance read at OD570.

### B. Taxane-resistant tumor cells in which resistance is mediated through tubulin

We observed the surprising finding that subject platinum-based compounds described herein were useful in inhibiting or killing tumour cells that were resistant to other chemotherapeutic agents, where such resistance is mediated through tubulin.

Tubulin-mutated cells (1A9-PTX10) were highly resistant to paclitaxel and taxotere - relative resistance in individual experiments ranged between 37 to 142-fold - yet remained susceptible to treatment with JM216, JM118 and JM383 - relative resistance in individual experiments between 0.9 to 3.9-fold. The parental non-mutated cell line,1A9, was used as control.

The tubulin mutated, paclitaxel-resistant human ovarian carcinoma cell line 1A9-PTX10 and its parental paclitaxel-sensitive cell line 1A9 were derived from the A2780 human ovarian carcinoma cell line (J Biol Chem (1997) 272, 17118-17124). 1,000-4,000 cells/well were exposed to the test compounds for 48 hours at various concentrations in order to calculate the IC50 values shown in Table 1, as above, and cytotoxicty was measured using the SRB assay according to Shekan et al. (J Natl Cancer Inst (1990) 82, 1107-112) (see Example 1A).

**Table 1. Cellular IC50's of Satraplatin and metabolites in Paclitaxel resistant cells**

| Compound | 1A9 ("non-resistant") | 1A9-PTX10 ("resistant") | | NCI-Adr resistant +verapamil ("non-resistant") | -verapamil ("resistant") | |
|---|---|---|---|---|---|---|
| | IC50 [µM] | IC50 [µM] | RR | IC50 [µM] | IC50 [µM] | RR |
| Paclitaxel | 0.005 +/- 0.003 (2) | 0,213 +/- 0.069 (2) | 42.6 | 0.031 (1) | 1.42 (1) | 46 |
| Taxotere | 0.002 +/- 0.000 (2) | 0.212 +/- 0.104 (2) | 105.8 | 0.019 (1) | 0.62 (1) | 33 |
| Satraplatin | 4.4 +/- 3.1 (3) | 5.7 +/- 1.6 (3) | 1.3 | 21.9 +/-0.0 (2) | 20.5 +/-1.6 (2) | 1.1 |
| (JM216) | | | | | | |
| JM118 | 0.5 +/- 0.2 (3) | 1.5 +/- 0.6 (3) | 3.1 | 2.3 +/- 1.3 (2) | 2.1 (1) | 1.1 |
| JM383 | 7.6 +/- 1.7 (3) | 16.0 +/- 5.7 (3) | 2.1 | | | |
| Cisplatin | 8.4 +/- 4.2 (3) | 13.9 +/- 5.2 (3) | 1.6 | 13.0 +/- 3.5 (2) | 13.2 +/- 2.6 (2) | 0.93 |
| Adriamycin | | | | 75 (1) | 0.65 (1) | 115 |

| | | | | | | |
|---|---|---|---|---|---|---|
| Legend: Table 1 shows the IC50 values as determined in the experiments described in Example 1. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e., the level of resistance conferred to the indicated drugs by the respective resistance mechanisms, and is calculated as the ratio of the means of the resistant cells compared to the sensitive cells. | | | | | | |

### COMPARATIVE EXAMPLE 2. Efficacy of satraplatin and its metabolites is maintained in campothecin-resistant tumor cells

In a comparative example, we observed the surprising finding that subject platinum-based compounds described herein, were useful in inhibiting or killing tumour cells that were resistant to other chemotherapeutic agents, where such resistance is mediated through topoisomerase I.

Topoisomerase-mutated cells (CEM/C2) were highly resistant to camptothecin - relative resistance in individual experiments ranged between 1,280 to 1,775-fold - yet remained susceptible to treatment with JM216, JM118 and JM383 - relative resistance in individual experiments between 0.48 to 0.82-fold. The parental non-mutated cell line, CEM, was used as control.

The Camptothecin resistant CEM/C2 cells were derived from the T lymphoblastoid leukemia cell line CCRF/CEM by selection in the presence of Camptohecin *in vitro* (Kapoor et al., 1995. Oncology Research 7; 83-95, ATCC

The effects of satraplatin and metabolites were evaluated in the CCRF/CEM and CEM/C2 cells using the Calcein AM viability assay (Molecular Probes) adapted for flow cytometry. The cells were plated in T-25 flasks in the presence or absence of compound and incubated for 48 hours before termination of the assay. Cells were washed twice with PBS and incubated in 200 µl of a solution of Calcein AM (0.3 nM)/PBS for 90 minutes at 37ºC. The cells were washed once with PBS before determining fluorescent units on a FACScan flow cytometer.

**Table 2. Cellular IC50's of Satraplatin and metabolites in Campothecin resistant cells**

| Compound | CEM | CEM/C2 | RR |
|---|---|---|---|
| | ("non-resistant") | ("resistant") | |
| | IC50 (µM) | IC50 (µM) | |
| Camptothecin | 0,005 +/- 0.001 (2) | 6.75 +/- 0.49 (2) | 1500 |
| Satraplatin | 5.06 +/- 1.97 (2) | 4.05 +/- 1.79 (2) | 0.80 |
| (JM216) | | | |
| JM118 | 0.43 +/- 0.22 (2) | 0.50 +/- 0.42 (2) | 1.18 |
| JM383 | 23.8 +/- 3.1 (2) | 12.2 +/- 0.5 (2) | 0.51 |
| Cisplatin | 2.60 +/- 1.07 (2) | 2.2 +/- 1.5 (2) | 0.85 |

| | | | |
|---|---|---|---|
| Legend: Table 2 shows the IC50 values as determined in the experiments described in Example 2. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e. the level of resistance conferred to the indicated drugs through atypical multi-drug resistance and mutant topoisomerase I. | | | |

### COMPARATIVE EXAMPLE 3. Efficacy of satraplatin and its metabolites is maintained in tumor cells in which resistance is mediated through ATP-binding cassette (ABC) transporters

We observed the surprising finding that subject platinum-based compounds described herein were useful in inhibiting or killing tumour cells that were resistant to other chemotherapeutic agents, where such resistance is mediated through ATP-binding cassette (ABC) transporters.

### A. Resistance mediated through BCRP (ABCG2)

The mitoxantrone-resistant colon carcinoma cell line HT29/MIT was used (Perego et al, Cancer Res, 61,6034). The development of drug resistance in this cell line is associated with the up-regulation of the breast carcinoma resistance protein (BCRP; ABCG2). The parental cell line, HT29, was used as a control.

2,000 cells/well were exposed to the test compounds for 48 hours at various concentrations in order to calculate the IC50 values shown in Table 3, as above, and cytotoxicty was measured using the SRB assay according to Shekan et al. (J Natl Cancer Inst (1990) 82, 1107-112) (see Example 1A). HT29/MIT cells were highly resistant to mitoxantrone - relative resistance in individual experiments ranged between 105 to 239-fold - yet remained susceptible to treatment with satraplatin and JM118 - relative resistance in individual experiments between 1.2 and 2.0-fold.

### B. Resistance mediated through MRP1 (ABCC1)

The etoposide-resistant breast cancer cell line MCF-7/VP was used (Schneider et al, Cancer Res, 54, 152). The development of drug resistance in this cell line is associated with the up-regulation of the multiple drug resistance protein 1 (MRP1; ABCC1; Schneider et al, Cancer Res, 54, 152; Perez-Soler et al, Int J Cancer, 71,35). The parental cell line, MCF-7, was used as a control.

2,000 cells/well were exposed to the test compounds for 48 hours at various concentrations in order to calculate the IC50 values shown in Table 3, as above, and cytotoxicty was measured using the SRB assay according to Shekan et al. (J Natl Cancer Inst (1990) 82, 1107-112) (see Example 1A). MCF-7/VP cells were highly resistant to etoposide - relative resistance in three individual experiments was determined to be >20-fold - yet remained susceptible to treatment with satraplatin, cisplatin and JM118 - relative resistance in individual experiments between 1.0 and 1.8-fold.

**Table 3. Cellular IC50's of Satraplatin and metabolites in mitoxantrone and etoposide resistant cells**

| Compound | HT-29 | HT-29/MIT | | MCF7 | MCF7/VP | |
|---|---|---|---|---|---|---|
| | ("non-resistant") | ("resistant") | | ("non-resistant") | ("resistant") | |
| | IC50 [µM] | IC50 [µM] | RR | IC50 [µM] | IC50 [µM] | RR |
| Mitoxantrone | 1.3 +/- 0.6 (3) | 198 +/-15 (3) | 175 +/-67 | | | |
| Etoposide | | | | 7.2 +/- 2.5 (3) | >100 (3) | >20 |
| Satraplatin | 6.9 +/- 2.8 (3) | 10.2 +/- 2.2 (3) | 1.6 +/- 0.3 | 3.0 +/- 1.2 (3) | 4.8 +/- 1.4 (3) | 1.6 +/- 0.2 |
| JM118 | 4.5 +/- 2.3 (4) | 6.0 +/- 3.3 (4) | 1.3 +/- 0.1 | 1.2 +/- 0.3 (3) | 1.3 +/- 0.3 (3) | 1.1 +/- 0.1 |
| Cisplatin | | | | 28.7 +/- 3.6 (3) | 31.2 +/- 7.1 (3) | 1.1 +/- 0.1 |

| | | | | | | |
|---|---|---|---|---|---|---|
| Table 3 shows the IC50 values as determined in the experiments described in Example 3. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e. the relative level of resistance conferred to the indicated drugs by the respective resistance mechanisms. | | | | | | |

### COMPARATIVE EXAMPLE 4. Efficacy of satraplatin and its metabolites is maintained in cisplatin-resistant tumor cells

In a comparative example, we observed the surprising finding that subject platinum-based compounds described herein were useful in inhibiting or killing tumour cells that were resistant to other platinum compounds, such as cisplatin.

The A129 cp80 cell line (received from Tito Fojo, NIH; Biochem Pharmacol 52, 1855), derived from the ovarian carcinoma A2780, was highly resistant to cisplatin - relative resistance in individual experiments ranged between 80 to 106-fold - yet remained susceptible to treatment with JM216, JM118 and JM383 - relative resistance in individual experiments between 0.19 to 2.59-fold (Table 1). The parental non-mutated cell line A 129 was used as control.

1,000-5,000 cells/well were exposed to the test compounds for 48 hours at various concentrations in order to calculate the IC50 values shown in Tables 3. Cytotoxicty was measured using the SRB assay according to Shekan et al. as described above in Example 1A.

**Table 4. Cellular IC50's of Satraplatin and metabolites in Cisplatin resistant cells**

| Compound | Cell line | | |
|---|---|---|---|
| | A129 | A129 cp80 | RR |
| | ("non-resistant") | ("resistant") | |
| | IC50 (µM) | IC50 (µM) | |
| Cisplatin | 0.13 +/- 0.06 (2) | 11.6 +/- 3.0 (2) | 89 |
| Satraplatin | 0.17 +/- 0.01 (2) | 1.13 +/- 0.18 (2) | 6.8 |
| (JM216) | | | |
| JM118 | 0.17 +/- 0.06 (2) | 0.43 +/- 0.33 (2) | 2.6 |
| JM383 | 1.29 +/- 0.11 (2) | 1.78 +/-1.15 (2) | 1.4 |

| | | | |
|---|---|---|---|
| Legend: Table 4 shows the IC50 values as determined in the experiments described in Example 4. Numbers in brackets indicate how often experiments were performed. In each single experiment a minimum of three replica wells was used for each drug concentration and cell line. Shown are mean values and standard deviations of the IC50s, as determined in the individual experiments. RR denominates the relative resistance, i.e. the relative level of resistance conferred to the indicated drugs by the mechanism that confers cisplatin resistance. | | | |

### References

R.D. Baird and S.B. Kaye, Drug resistance reversal - are we getting closer?, European Journal of Cancer, 39: 2450-61 (2003).
P. Giannokakou et al., J. Biol. Chem. 272: 17118-125 (1997).
P. Giannokakou et al., Proc. Natl. Acad. Sci. U.S.A. 97: 2904-2909 (2000)
Kelland L R. An update on satraplatin: the first orally available platinum anticancer drug. Expert Opin Investig Drugs 9(6):1373-1382, 2000.

## Claims

1. Use of a compound of the structure 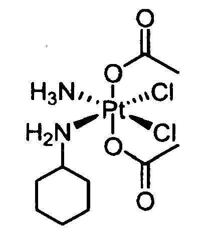 for the preparation of a pharmaceutical composition for the treatment of a cancer or tumor resistant or refractory to a taxane.

2. The use according to claim 1, wherein said treatment results in killing or inhibition of the growth of tumor cells.

3. The use according to claim 1 or 2, wherein the resistance or refractoriness of said cancer or tumor is mediated through tubulin.

4. The use according to claim 1 or 2, wherein the resistance or refractoriness of said cancer or tumor is mediated through multidrug resistance.

5. The use according to claim 4, wherein said multidrug resistance is mediated through overexpression of an ABC transporter.

6. The use according to any one of claims 1-5, wherein said taxane is paclitaxel.

7. The use according to any one of claims 1-5, wherein said taxane is docetaxel.

8. The use according to any one of claims 1-7, wherein said tumor comprises a solid tumor.

9. The use of claim 8, wherein said solid tumor is selected from: breast cancer, cervical cancer, colorectal cancer, peritoneal cancer, ovarian cancer, bronchial cancer, small cell lung cancer, non-small cell lung cancer, gastric, prostate, and head and neck cancer, or metastases thereof.

10. The use of claim 9, wherein said solid tumor is prostate cancer.

11. The use of claim 9, wherein said solid tumor is breast cancer.

12. The use of claim 9, wherein said solid tumor is ovarian cancer.

13. The use of claim 9, wherein said solid tumor is non-small cell lung cancer.

14. The use according to any one of claims 1-7, wherein said tumor comprises a hematological tumor.

15. The use according to any one of claims 1-14, wherein said treatment includes the further administration of an effective amount of another pharmaceutical ingredient.

16. The use of claim 15, wherein said other pharmaceutical ingredient is an anti-emetic or anti-diarrheal therapeutic composition

17. The use of claim 15, wherein said other pharmaceutical ingredient is an agent that overcomes a specific drug resistance mechanism.

18. The use of claim 15, wherein said other pharmaceutical ingredient is another anti-cancer therapeutic agent.

## Patentansprüche

1. Verwendung einer Verbindung mit der Struktur 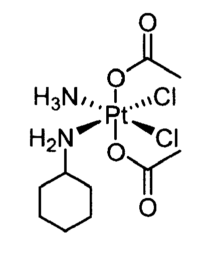 zur Herstellung einer pharmazeutischen Zusammensetzung zur Behandlung einer Krebserkrankung oder eines Tumors, welche gegenüber einem Taxan resistent oder widerstandsfähig sind.

2. Verwendung nach Anspruch 1, wobei die Behandlung zur Abtötung oder Inhibierung des Wachstums von Tumorzellen führt.

3. Verwendung nach Anspruch 1 oder 2, wobei die Resistenz oder Widerstandsfähigkeit der Krebserkrankung oder des Tumors durch Tubulin vermittelt wird.

4. Verwendung nach Anspruch 1 oder 2, wobei die Resistenz oder Widerstandsfähigkeit der Krebserkrankung oder des Tumors durch Multidrug Resistance vermittelt wird.

5. Verwendung nach Anspruch 4, wobei die Multidrug Resistance durch Überexpression eines ABC-Transporters vermittelt wird.

6. Verwendung nach einem der Ansprüche 1-5, wobei das Taxan Paclitaxel ist.

7. Verwendung nach einem der Ansprüche 1-5, wobei das Taxan Docetaxel ist.

8. Verwendung nach einem der Ansprüche 1-7, wobei der Tumor einen soliden Tumor umfasst.

9. Verwendung nach Anspruch 8, wobei der solide Tumor ausgewählt ist aus: Brustkrebs, Gebärmutterhalskrebs, Kolorektralkrebs, Peritonealkrebs, Eierstockkrebs, Bronchialkrebs, kleinzelliger Lungenkrebs, nicht-kleinzelliger Lungenkrebs, Magen-, Prostata- und Kopf- und Halskrebs oder Metastasen davon.

10. Verwendung nach Anspruch 9, wobei der solide Tumor Prostatakrebs ist.

11. Verwendung nach Anspruch 9, wobei der solide Tumor Brustkrebs ist.

12. Verwendung nach Anspruch 9, wobei der solide Tumor Eierstockkrebs ist.

13. Verwendung nach Anspruch 9, wobei der solide Tumor nicht-kleinzelliger Lungenkrebs ist.

14. Verwendung nach einem der Ansprüche 1-7, wobei der Tumor einen hämatologischen Tumor umfasst.

15. Verwendung nach einem der Ansprüche 1-14, wobei die Behandlung die weitere Verabreichung einer wirksamen Menge eines anderen pharmazeutischen Inhaltsstoffes beinhaltet.

16. Verwendung nach Anspruch 15, wobei der andere pharmazeutische Inhaltsstoff eine therapeutische Zusammensetzung gegen Erbrechen oder Durchfall ist.

17. Verwendung nach Anspruch 15, wobei der andere pharmazeutische Inhaltsstoff ein Mittel ist, das einen spezifischen Arzneimittelresistenzmechanismus überwindet.

18. Verwendung nach Anspruch 15, wobei der andere pharmazeutische Inhaltsstoff ein weiteres therapeutisches Antikrebsmittel ist.

## Revendications

1. Utilisation d'un composé de structure 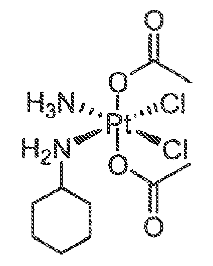 pour la préparation d'une composition pharmaceutique destinée au traitement d'un cancer ou d'une tumeur résistant ou réfractaire à un taxane.

2. Utilisation suivant la revendication 1, dans laquelle ledit traitement a pour résultat la destruction ou l'inhibition de la croissance des cellules tumorales.

3. Utilisation suivant la revendication 1 ou 2, dans laquelle la résistance ou le caractère réfractaire dudit cancer ou de ladite tumeur est soumis à une médiation par la tubuline.

4. Utilisation suivant la revendication 1 ou 2, dans laquelle la résistance ou le caractère réfractaire dudit cancer ou de ladite tumeur est soumis à une médiation par la résistance à des médicaments multiples.

5. Utilisation suivant la revendication 4, dans laquelle ladite résistance à des médicaments multiples est soumise à une médiation par la surexpression d'un transporteur ABC.

6. Utilisation suivant l'une quelconque des revendications 1 à 5, dans laquelle ledit taxane est le paclitaxel.

7. Utilisation suivant l'une quelconque des revendications 1 à 5, dans laquelle ledit taxane est le docétaxel.

8. Utilisation suivant l'une quelconque des revendications 1 à 7, dans laquelle ladite tumeur comprend une tumeur solide.

9. Utilisation suivant la revendication 8, dans laquelle ladite tumeur solide est choisie entre : le cancer du sein, le cancer du col de l'utérus, le cancer colorectal, le cancer du péritoine et le cancer des ovaires, le cancer bronchique, le cancer pulmonaire à petites cellules, le cancer pulmonaire non à petites cellules, le cancer gastrique, le cancer de la prostate et le cancer de la tête et du cou ou leurs métastases.

10. Utilisation suivant la revendication 9, dans laquelle ladite tumeur solide est le cancer de la prostate.

11. Utilisation suivant la revendication 9, dans laquelle ladite tumeur solide est le cancer du sein.

12. Utilisation suivant la revendication 9, dans laquelle ladite tumeur solide est le cancer des ovaires.

13. Utilisation suivant la revendication 9, dans laquelle ladite tumeur solide est le cancer pulmonaire non à petites cellules.

14. Utilisation suivant l'une quelconque des revendications 1 à 7, dans laquelle ladite tumeur comprend une tumeur hématologique.

15. Utilisation suivant l'une quelconque des revendications 1 à 14, dans laquelle ledit traitement comprend l'administration supplémentaire d'une quantité efficace d'un autre ingrédient pharmaceutique.

16. Utilisation suivant la revendication 15, dans laquelle ledit autre ingrédient pharmaceutique est une composition thérapeutique antiémétique ou antidiarrhéique.

17. Utilisation suivant la revendication 15, dans laquelle ledit autre ingrédient pharmaceutique est un agent qui surmonte un mécanisme spécifique de résistance à un médicament.

18. Utilisation suivant la revendication 15, dans laquelle ledit autre ingrédient pharmaceutique est un autre agent thérapeutique anticancéreux.
